# EUROPÄISCHE PATENTSCHRIFT

(11) **EP 1 673 334 B1**
(45) Veröffentlichungstag und Bekanntmachung des Hinweises auf die Patenterteilung: **14.12.2011**
(21) Anmeldenummer: 04765776.2
(22) Anmeldetag: 02.10.2004
(51) Int. Cl.: C07C 51/44

(54) **VERFAHREN DER REKTIFIKATIVEN AUFTRENNUNG EINER ACRYLSÄURE EN THALTENDEN FLÜSSIGKEIT**
METHOD FOR RECTIFICATION SEPARATION OF A LIQUID FROM AN ACRYLIC ACID
PROCEDE DE SEPARATION RECTIFICATIVE D'UN LIQUIDE CONTENANT DE L'ACIDE ACRYLIQUE

(30) Priorität: 09.10.2003 US 509540 P; 09.10.2003 DE 10347664
(43) Veröffentlichungstag der Anmeldung: 28.06.2006
(73) Patentinhaber: BASF SE, 67056 Ludwigshafen (DE)
(72) Erfinder: HÖFER, Frank, 67071 Ludwigshafen (DE); SCHLIEPHAKE, Volker, 67105 Schifferstadt (DE); MÜLLER-ENGEL, Klaus Joachim, 76297 Stutensee (DE)
(86) Internationale Anmeldenummer: PCT/EP2004/011026
(87) Internationale Veröffentlichungsnummer: WO 2005/035478

(56) Entgegenhaltungen:
- EP-A- 0 000 110
- WO-A-98/08798
- WO-A-02/076917
- WO-A-2004/035514

## Beschreibung

Vorliegende Erfindung betrifft ein Verfahren der rektifikativen Auftrennung einer Acrylsäure enthaltenden Flüssigkeit F, bei dem man die Acrylsäure enthaltende Flüssigkeit F über eine Zuführstelle Z einer Rektifikationskolonne zuführt und oberhalb der Zuführstelle Z der Rektifikationskolonne an einer Entnahmestelle E einen Stoffstrom S entnimmt, dessen Gehalt an Acrylsäure, bezogen auf das Gewicht des Stoffstroms S, ≥ 90 Gew.-% beträgt und größer ist als der entsprechende Acrylsäuregehalt der Flüssigkeit F in Gew.-%.

Unter einer Flüssigkeit F an der Zuführstelle Z soll ein Stoffstrom verstanden werden, der zu mehr als 80 %, vorzugsweise zu mehr als 85 %, oder zu mehr als 90 %, oder zu mehr als 95 %, oder zu mehr als 99 % seines Gesamtvolumens als kondensierte Phase vorliegt. D.h., die Verfahren der fraktionierenden oder der totalen Kondensation wie sie z.B. in der DE-A 19924532 oder in der DE-A 19924533 beschrieben sind, sollen von der vorliegenden Erfindung nicht umfasst werden.

Acrylsäure ist aufgrund ihrer sehr reaktionsfähigen Doppelbindung sowie ihrer Säurefunktion ein wertvolles Monomer zur Herstellung von Polymerisaten, die z.B. als wasserabsorbierende Harze verwendbar sind.

Unter anderem ist Acrylsäure durch heterogen katalysierte partielle Gasphasenoxidation von Propen, Propan und/oder Acrolein mit Sauerstoff oder Sauerstoff enthaltenden Gasen in Gegenwert von festen Katalysatoren und bei erhöhter Temperatur zugänglich (vgl. z.B. EP-A 700714).

Bei diesem Verfahren wird jedoch nicht reine Acrylsäure sondern ein Produktgasgemisch erhalten, das neben Acrylsäure als Nebenkomponenten nicht umgesetztes Acrolein und/oder Propen sowie Bestandteile wie Wasserdampf, Kohlenoxide (CO, CO₂), Stickstoff, Sauerstoff, Propan, Methan, niedere gesättigte Carbonsäuren wie Ameisensäure, Essigsäure und Propionsäure, niedere Aldehyde wie Formaldehyd, Benzaldehyd, Acrolein und Furfurale sowie höhere Carbonsäuren bzw. deren Anhydride wie Benzoesäure, Phthalsäureanhydrid und Maleinsäureanhydrid enthält.

Die Grundabtrennung der Acrylsäure aus dem gasförmigen Produktgasgemisch erfolgt in der Regel durch Absorption in ein Lösungsmittel (z.B. Wasser oder ein organisches Lösungsmittel) oder durch fraktionierende Kondensation des Produktgasgemischs. Das dabei anfallende Kondensat bzw. Absorbat wird nachfolgend rektifikativ (in der Regel in mehreren Stufen) unter Erhalt von mehr oder weniger reiner Acrylsäure aufgetrennt (vgl. WO 03/051810, EP-A 982289, EP-A 982287, DE-A 19606877 und

DE-A 10224341). Anstelle der fraktionierenden Kondensation kann auch zunächst eine Totalkondensation angewandt und das dabei anfallende Kondensat anschließend rektifikativ aufgetrennt werden.

Nachteilig an einer rektifikativen Auftrennung einer Acrylsäure enthaltenden Flüssigkeit ist, dass es sich um ein thermisches Trennverfahren handelt, bei dem die angestrebte Auftrennung der Zufuhr von thermischer Energie bedarf. Die ist insofern nachteilig, als in flüssiger Phase befindliche Acrylsäure insbesondere bei Einwirkung von thermischer Energie zu ungewollter radikalischer Polymerisation neigt.

Trotz der Mitverwendung von radikalischen Polymerisationsinhibitoren (z.B. Phenothizin, Monomethylether des Hydrochinons, Hydrochinon, N-Oxyl-Radikale, etc.) müssen deshalb rektifikative Auftrennungen von Acrylsäure enthaltenden Flüssigkeiten von Zeit zu Zeit unterbrochen werden, um während der Rektifikation in unerwünschter Weise gebildetes Polymerisat aus der Rektifikationskolonne (insbesondere in deren oberem Teil) zu entfernen (vgl. z.B. DE-A 19746688, DE-A 10211273, DE-A 19536179, EP-A 1033359 und DE-A 10213027).

Solche Betriebsabstellungen von Rektifikationskolonnen sind jedoch gleichbedeutend mit Produktionsverlust, weshalb weltweit enorme Anstrengungen unternommen werden, um verbesserte Polymerisationsinhibitorsysteme aufzufinden, die einen verlängerten Betrieb rektifikativer Auftrennungen von Acrylsäure enthaltenden Flüssigkeiten ermöglichen.

Letzteres gilt insbesondere für denjenigen Bereich der Rektifikationskolonne, dei oderhalb der Zulaufstelle der rektifikativ aufzutrennenden, Acrylsäure enthaltenden, Flüssigkeit in die Rektifikationskolonne liegt. Dies u.a. deshalb, weil sich in diesem Bereich diejenigen Nebenkomponenten anreichern, die leichter flüchtig als Acrylsäure sind. Verschiedene dieser vergleichweise leichtflüchtigen Nebenkomponenten (z.B. Acrolein) fördern jedoch die radikalische Polymerisationsneigung von Acrylsäure (vgl. z.B. EP-A 1041062), weshalb deren Inhibierung in diesem Bereich von besonderer Bedeutung ist.

Nachteilig an den bekannten Verfahren der rektifikativen Auftrennung einer Acrylsäure enthaltenden Flüssigkeit, bei dem man die Acrylsäure enthaltende Flüssigkeit einer Rektifikationskolonne zuführt und oberhalb der Zuführstelle der Rektifikationskolonne einen Stoffstrom entnimmt, dessen Gehalt an Acrylsäure ≥ 90 Gew.-% beträgt und größer ist als der entsprechende Acrylsäuregehalt der Flüssigkeit, ist jedoch, dass die Polymerisationsinhibierung im oberen Teil der Rektifikationskolonne nicht im vollen Umfang zu befriedigen vermag. Die Aufgabe der vorliegenden Erfindung bestand daher darin, diese Polymerisationsinhibierung zu verbessern.

Eine weitere, normalerweise unerwünschte, Begleiterscheinung von in kondensierter Phase befindlicher Acrylsäure ist die Ausbildung von Oligomeren mit ≥ 2 Monomereinheiten durch Michael-Addition von Acrylsäure an sich selbst sowie an das sich dabei bildende Acrylsäure-Dimere (Oligomere). Aus statistischen Gründen ist dabei vor allem die Bildung von Diacrylsäure 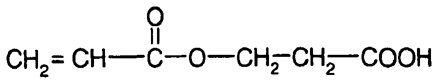 von Bedeutung, wohingegen die Bildung höherer Acrylsäure-Oligomere (Trimere, Tetramere, etc.) im allgemeinen vernachlässigbar ist.

Da Diacrylsäure eine weniger ausgeprägte Neigung zur radikalischen Polymerisation als Acrylsäure aufweist, unterliegt Acrylsäure üblicherweise dem Erfordernis, möglichst wenig Diacrylsäure zu enthalten (vgl. DE-A 19923389, DE-A 19627679 und WO-03/064367). Dies vor allem auch deshalb, als die Diacrylsäurebildung reversibel ist und im Fall radikalisch copolymerisierter Diacrylsäure bei nachträglicher thermischer Behandlung des Polymerisats (z.B. bei der Trocknung eines wasserabsorbierenden Harzes) monomere Acrylsäure abgespalten werden kann, was in der Regel unerwünscht ist.

Eine Abtrennung der Diacylsäure von Acrylsäure ist aufgrund der signifikant verschiedenen Siedepunkte rektifikativ in einfacher Weise möglich. Diacrylsäure reichert sich im unteren Teil der Rektifikationskolonne an und Acrylsäure im oberen Teil der Rektifikaitonskolonne, der an Diacrylsäure normalerweise im wesentlichen weitgehend frei ist. Dies vor allem auch deshalb, weil die Neubildung von Diacrylsäure ein langsamer Prozess ist.

Überlässt man Acrylsäure bei Normalbedingungen sich selbst, so benötigt eine Neubildung von 150 ppm Diacrylsäure, bezogen auf das Gewicht der Acrylsäure, etwa 24 h. Ein Vorhandensein von Diacrylsäure im oberen Teil der Rektifikationskolonne ist infolge des Vorgenannten bisher unerwünscht.

Als Grundlage der vorliegenden Erfindung wurde jedoch überraschend gefunden, dass ein Beisein von Diacrylsäure bezüglich einer radikalischen Polymerisation von Acrylsäure polymerisationsinhibierend wirkt. Dies wird darauf zurückgeführt, dass Diacrylsäure eine deutlich weniger ausgeprägte Neigung zur radikalischen Polymerisation aufweist als Acrylsäure selbst. Vermutlich wirkt in Acrylsäure verteilte Diacrylsäure infolge dieser verminderten Polymerisationsneigung auf radikalisch waschsende Acrylsäurepolymerisatketten wie ein Wachstumskettenabbrecher.

Als Lösung der Aufgabe der vorliegenden Erfindung wurde daher ein Verfahren der rektifikativen Auftrennung einer Acrylsäure enthaltenden Flüssigkeit F, bei dem man die Acrylsäure enthaltende Flüssigkeit F über eine Zuführstelle Z einer Rektifikationskolonne zuführt und oberhalb der Zuführstelle Z der Rektifikationskolonne an einer Entnahmestelle E einen Stoffstrom S entnimmt, dessen Gehalt an Acrylsäure, bezogen auf das Gewicht des Stoffstroms S, ≥ 90 Gew.-% beträgt und größer ist als der entsprechende Acrylsäuregehalt der Flüssigkeit F in Gew.-%, gefunden, das dadurch gekennzeichnet ist, dass innerhalb des wenigstens zwei theoretische Trennstufen oberhalb der Zuführstelle Z befindlichen Bereichs der Rektifikationskolonne der Gehalt G der Rücklaufflüssigkeit an Diacrylsäure, bezogen auf das Gewicht der Rücklaufflüssigkeit, wenigstens in Teilbereichen ≥ 550 Gew.-ppm beträgt.

Innerhalb einer Rektifikationskolonne werden absteigende Flüssigkeitsphase (Rücklaufflüssigkeit) und aufsteigende Dampfphase im Gegenstrom zueinander geführt. Infolge des zwischen den Stoffströmen bestehenden Ungleichgewichts findet ein Wärmeund Stoffaustausch statt, der die gewünschte Stoffauftrennung bedingt. In der Regel befinden sich zur Erhöhung der Stoffaustauschfläche trennwirksame Einbauten in einer Rektifikationskolonne. Als solche Einbauten kommen, auch im vorliegenden Verfahren, z.B. Packungen, Füllkörperschüttungen und/oder Stoffaustauschböden jedweder Art in Betracht.

Stoffaustauschböden, auf denen Gleichgewicht herrscht zwischen ablaufender Flüssigkeit und aufsteigendem Dampf werden als theoretische Böden bezeichnet. Dieser Degriff lässt sich auf alle anderen für Gegenstromrektifikationen geeigneten trennwirksamen Einbauten übertragen (z.B. Packungen und Füllkörperschüttungen).

In dieser Schrift wird deshalb in zweckmäßiger Weise ganz allgemein von theoretischer Trennstufe gesprochen. Dabei wird als theoretische Trennstufe diejenige Raumeinheit definiert, die eine Anreicherung entsprechend dem thermodynamischen Gleichgewicht bewirkt.

Das erfindungsgemäße Verfahren erweist sich insbesondere dann als vorteilhaft, wenn der Acrylsäuregehalt des Stoffstroms S ≥ 93 Gew.-% oder ≥ 95 Gew.-% beträgt. Es ist aber auch dann vorteilhaft anwendbar, wenn der Acrylsäuregehalt des Stoffstroms S ≥ 97 Gew.-%, oder ≥ 98 Gew.-%, oder ≥ 99 Gew.-%, oder 99,5 Gew.-% oder ≥ 99,8 Gew.% beträgt.

Bevorzugt liegt beim erfindungsgemäßen Verfahren der Gehalt der Rücklaufflüssigkeit an Diacrylsäure, bezogen auf das Gewicht der Rücklaufflüssigkeit, innerhalb des wenigstens zwei (bzw. drei, oder vier, oder fünf) theoretische Trennstufen oberhalb der Zufuhrstelle Z befindlichen Bereichs der Rektifikationskolonne wenigstens in Teilbereichen nicht nur bei Werten von ≥ 550 Gew.-ppm, sondern bei Werten von ≥ 600 Gew.-ppm, oder bei Werten von ≥ 650 Gew.-ppm, oder bei Werten von ≥ 700 Gew.-ppm, oder bei Werten von ≥ 750 Gew.-ppm, oder bei Werten von ≥ 800 Gew.-ppm.

Ganz besonders bevorzugt beträgt dieser Diacrylsäuregehalt G beim erfindungsgemäßen Verfahren ≥ 850 Gew.-ppm, oder ≥ 900 Gew.-ppm, oder ≥ 950 Gew.-ppm, oder ≥ 1000 Gew.-ppm.

Noch vorteilhafter liegt der vorgenannte Diacrylsäuregehalt G beim erfindungsgemäßen Verfahren bei Werten von ≥ 1250 Gew.-ppm, oder ≥ 1500 Gew.-ppm, oder ≥ 1750 Gew.-ppm, oder ≥ 2000 Gew.-ppm, bzw. ≥ 2500 Gew.-ppm, oder ≥ 3000 Gew.-ppm.

In der Regel wird der vorgenannte Diacrylsäuregehalt jedoch nicht mehr als 10 oder 5 Gew.-% betragen und normalerweise bei Werten von ≤ 3 Gew.-% bzw. ≤ 2 Gew.-% liegen.

Erfindungsgemäß zweckmäßig sind die vorgenannten erfindungsgemäß günstigen Diacrylsäuregehalte G der Rücklaufflüssigkeit wenigstens in jenem wenigstens zwei theoretische Trennstufen oberhalb der Zuführstelle Z befindlichen Bereich der Rektifikationskolonne gegeben, der, ausgehend von der Entnahmestelle E des Stoffstroms S, im Intervall von E + 0,5 theoretische Trennstufen bis E + 5 theoretische Trennstufen liegt.

Besonders günstig ist das erfindungsgemäße Verfahren dann, wenn die vorgenannten erfindungsgemäß günstigen Diacrylsäuregehalte G der Rücklaufflüssigkeit im gesamten wenigstens zwei, bzw. wenigstens vier, bzw. wenigstens sechs, bzw. wenigstens acht oder mehr theoretische Trennstufen oberhalb der Zuführstelle Z befindlichen Bereich der Rektifikationskolonne gegeben sind.

Erfindungsgemäß vorteilhaft ist das erfindungsgemäße Verfahren jedoch bereits dann, wenn sich die Entnahmestelle E wenigstens zwei, bzw. wenigstens vier, bzw. wenigstens sechs, bzw. wenigstens acht oder mehr theoretische Trennstufen oberhalb der Zuführstelle Z befindet und die vorgenannten Diacrylsäuregehalte G der Rücklaufflüssigkeit im gesamten oberhalb der Entnahmestelle E befindlichen Bereich der Rektifikationskolonne gegeben sind.

Erfindungsgemäß zweckmäßig befindet sich die Entnahmestelle E beim erfindungsgemäßen Verfahren wenigstens 2 theoretische Trennstufen, oder wenigstens 5 theoretische Trennstufen, oder wenigstens 8 theoretische Trennstufen, oder wenigstens 10 theoretische Trennstufen, oder wenigstens 12 theoretische Trennstufen oberhalb der Zuführstelle Z der Rektifikationskolonne.

Günstige erfindungsgemäße Verfahren der rektifikativen Auftrennung weisen darüber hinaus oberhalb der Entnahmestelle E wenigstens 0,5 theoretische Trennstufen, oder wenigstens 1 theoretische Trennstufe, oder wenigstens 1,5 theoretische Trennstufen auf.

Im beim erfindungsgemäßen Verfahren entnommenen Stoffstrom S gegebenenfalls enthaltene Diacrylsäure kann bei Bedarf in nachfolgenden Trennstufen abgetrennt werden. Diese Trennstufen können z.B. kristallisativer aber auch rektifikativer Natur sein. Letzteres u.a. dann, wenn die Rektifikation unter solchen Bedindungen durchgeführt wird, bei denen die Polymerisationsneigung der Acrylsäure minimiert ist und keine Zusatzinhibierung mittels in der Rücklaufflüssigkeit enthaltener Diacrylsäure erforderlich ist. Die dabei abgetrennte Diacrylsäure kann in an sich bekannter Weise (vgl. z.B. WO 03/048100) in Acrylsäure rückgespalten werden.

Die erfindungsgemäß erforderliche Einstellung des Diacrylsäuregehalts der Rücklaufflüssigkeit kann auf unterschiedliche Art und Weise realisiert werden. Zum einen kann der Rücklaufflüssigkeit (z.B. am Kopf der Kolonne) eine von Acrylsäure verschiedene Brönsted-Säure zugesetzt werden, die die Michael-Addition der Acrylsäure katalysiert. Erfindungsgemäß zweckmäßig beträgt die Säurestärke solcher Brönsted-Säuren, angegeben in Gestalt ihres pKₛ-Wertes (bei 25°C, 1 atm, Wasser als Lösungsmittel; vgl. Grundlagen der allgemeinen und anorganischen Chemie, H.R. Christen, Verlag Sauerländer, Aarau, 1973, S. 354) ≤ 16, vorzugsweise ≤ 7, besonders bevorzugt ≤ 5.

Erfindungsgemäß besonders geeignet sind z.B. H₂O und starke protische Mineralsäuren wie H₂SO₄ HCl oder H₃PO₄. Besonders bevorzugt sind unter diesen leicht flüchtige Mineralsäuren (bzw. insbesondere leichter als Acrylsäure flüchtige, d.h., tiefer als Acrylsäure siedende Brönsted-Säuren mit pKₛ ≤ 16). Im unteren Teil der Rektifikationskolonne kann die katalytische Wirkung der Mineralsäure durch Zusatz einer neutralisierenden Brönsted-Base wieder aufgehoben werden.

Anstelle einer Brönsted-Säure kann aber auch eine die Diacrylsäurebildung katalysierende Brönsted-Base zugesetzt werden. Erfindungsgemäß zweckmäßig beträgt die Basenstarke solcher Brönsted-Basen, angegeben in Gestalt ihres pK_{b}-Wertes (bei 25°C, 1 atm, Wasser als Lösungsmittel; vgl. Grundlagen der organischen Chemie, H.R. Christen, Verlag Sauerländer, Aarau, 1975, S. 392) ≤ 10, vorzugsweise ≤ 8 und besonders bevorzugt ≤ 5. Erfindungsgemäß besonders geeignet sind z.B. aminische Brönsted-Basen wie Methylamin, Dimethylamin, Trimethylamin, Ethylamin, Diethylamin, Triethylamin, Tetramethylethylendiamin, n-Propylamin, n-Butylamin, Benzylamin, Pyridin, Anilin, Harnstoff oder Ammoniak. Besonders bevorzugt sind unter diesen leicht flüchtige aminische Brönsted-Basen. Selbstverständlich können auch entsprechende wässrige Lösungen am Kolonnenkopf zugesetzt werden. Im unteren Teil der Rektifikationskolonne kann die katalytische Wirkung der Brönsted-Base durch Zusatz einer neutralisierenden Brönsted-Säure wieder aufgehoben werden. Gegebenenfalls findet jedoch zum unteren Teil der Rektifikationskolonne hin eine automatische Selbstaufhebung der katalytischen Wirkung der Brönsted-Base statt, in dem diese bei dem nach unten in der Rektifikationskolonne zunehmenden Temperaturen mit Acrylsäure zu den entsprechenden Amiden reagieren und sich im Kolonnensumpf anreichern.

Zusätzlich oder alternativ kann der Zusatz, z.B. der wässrige, auch bereits in die aufzutrennende Flüssigkeit F hinein erfolgen. Dies gilt insbesondere für den Fall von leichter als Acrylsäure flüchtigen Brönsted-Säuren oder Basen. Wird die Flüssigkeit F durch Absorption von Acrylsäure aus Reaktionsgasgemischen aus Partialoxidationen erzeugt, kann der Zusatz bereits im Verlauf der Absorption erfolgen.

Selbstverständlich kann beim erfindungsgemäßen Verfahren die erforderliche Diacrylsäure der Rücklaufflüssigkeit aber auch als solche zugesetzt werden. Diacrylsäure ist z.B. dadurch erhältlich, dass man sie zunächst in Acrylsäure anreichert und nachfolgend auf destillative oder kristallisative Weise abtrennt. Anstelle abgetrennte Diacrylsäure zuzusetzen, kann auch die hochgradig Diacrylsäure enthaltende Acrylsäure selbst im oberen Rektifikationskolonnenteil der Rücklaufflüssigkeit zugesetzt werden. In anwendungstechnisch zweckmäßiger Weise wird man dieser Acrylsäure vorteilhaft auch in der Rektifikationskolonne benötigten sonstigen Polymerisationsinhibitor zusetzen. Selbstredend wird man die Diacrylsäure in diesem Fall in einer Acrylsäure anreichert, deren Reinheitsgrad, abgesehen von gegebenenfalls enthaltenem Inhibitor, demjenigen des beim erfindungsgemäßen Verfahren abzutrennenden Stoffstroms S wenigstens entspricht.

Natürlich wird man das erfindungsgemäße Verfahren unter Zusatz der in an sich bekannter Weise zuzusetzenden sonstigen Polymerisationsinhibitoren wie z.B. Phenothiazin, Monomethylether des Hydrochinons, N-Oxyl-Radikale etc. durchführen. Vorzugsweise wird man diese am Kolonnenkopf in gelöster (z.B. in Diacrylsäure enthaltender Acrylsäure) oder in geschmolzener Form zusetzen. Allerdings kann beim erfindungsgemäßen Verfahren deren Verbrauchsmenge verringert werden.

Das erfindungsgemäße Verfahren eignet sich insbesondere dann, wenn die Rektifikationskolonne eine Bodenkolonne ist. Dies ist eine Rektifikationskolonne, die als trennwirksame Einbauten im wesentlichen ausschließlich Stoffaustauschböden enthält. Erfindungsgemäß zweckmäßig sind dabei solche Bodenkolonnen, deren Stoffaustauschböden zu wenigstens 80 % ihrer Anzahl Siebböden sind. Vorteilhaft enthält die erfindungsgemäß einzusetzende Boden-Rektifikationskolonne ausschließlich Siebböden als Stoffaustauschböden. Darunter sollen in dieser Schrift Platten verstanden werden, die als Durchtrittsstellen für die aufsteigende Gas- bzw. Dampfphase (die Begriffe "gasförmig" und "dampfförmig" werden in dieser Schrift synonym verwendet) einfache Löcher und/oder Schlitze aufweisen.

Die zu verwendenden Siebböden werden dabei üblicherweise in zwei Gruppen differenziert, nämlich in solche mit Flüssigkeitszwangsführung und solche ohne Flüssigkeitszwangsführung.

Ganz generell wird Flüssigkeitszwangsführung bei Stoffaustauschböden dadurch erzielt, dass die Stoffaustauschböden wenigstens einen Ablaufschacht aufweisen (Ablauf), durch den die Flüssigkeit unabhängig vom Strömungsweg des Dampfes vom höher gelegenen Boden auf den tiefer gelegenen Boden fließt (Zulauf). Die horizontale Flüssigkeitsströmung über den Austauschboden vom Zulauf zum Ablauf wird entsprechend der verfahrenstechnischen Aufgabenstellung gewählt. Das Gas bzw. der Dampf tritt durch die offenen Querschnitte der Bodenplatte.

Wird die Flüssigkeit im Umkehrstrom über den Boden geführt (Zulauf und Ablauf des Stoffaustauschbodens sind auf der gleichen Seite des Bodens angeordnet), spricht man von Umkehrstromböden. Bei Radialstromböden strömt die Flüssigkeit auf dem Boden radial von der Mitte (Zulauf) zum Ablauf am Rand des Bodens.

Bei den Querstromböden wird die Flüssigkeit, über den gesamten Fließbereich betrachtet, quer über den Boden vom Zulauf zum Ablauf geführt. In der Regel sind Querstromböden einflutig gestaltet. D.h., Zulauf und Ablauf sind auf gegenüberliegenden Seiten des Bodens angeordnet. Sie können aber auch zweiflutig (oder auch mehr als zweiflutig) gestaltet sein. In diesem Fall kann der Zulauf z.B. in der Mitte und je ein Ablauf auf gegenüberliegenden Seiten des Stoffaustauschbodens angeordnet sein.

D.h., bei Siebböden wird die Flüssigkeitszwangsführung dadurch erzielt, dass die Siebböden neben den Durchtrittsstellen für die aufsteigende Gas- bzw. Dampfphase wenigstens einen Ablaufschacht aufweisen (Ablauf), durch den die Flüssigkeit unabhängig vom Strömungsweg des Dampfes vom höher gelegenen Boden auf den nächsten tiefer gelegenen Boden fließt (Zulauf). Die Flüssigkeit fließt z.B. im Querstrom über den Boden vom wenigstens einen Zulauf zum wenigstens einen Ablauf, wobei das Zulaufund Ablaufrohr den Flüssigkeitsverschluss und die gewünschte Flüssigkeitshöhe auf dem Boden garantieren. Häufig (insbesondere bei geringen Kolonnendurchmessern) sind die Siebböden mit Flüssigkeitszwangsführung einflutig gestaltet. D.h., Zulauf und Ablauf sind auf gegenüberliegenden Seiten des Bodens angeordnet. Sie können aber auch zweiflutig (oder auch mehr als zweiflutig) gestaltet sein. In diesem Fall kann der Zulauf z.B. in der Mitte und je ein Ablauf auf gegenüberliegenden Seiten des Stoffaustauschbodens angeordnet sein. Nachfolgend sollen solche Siebböden als Zwangssiebböden bezeichnet werden. Bei ihnen wird ein die Trennwirkung minderndes Durchregnen der Flüssigkeit nicht wie beim hydraulisch abgedichteten Querstromboden durch Kamine verhindert, in die die Durchtrittsöffnungen fortführen, sondern es bedarf dazu einer minimalen Dampfbelastung. Der Dampf tritt aufsteigend durch die Durchtrittsöffnungen und durchperlt die vom Ablaufrohr gehaltene Flüssigkeitsschicht.

Von den Zwangssiebböden unterscheiden sich die Dual-Flow- oder auch Regensiebböden dadurch, dass sie kein Ablaufsegment enthalten. Durch die Abwesenheit von Ablaufsegmenten (Ablaufschächten) treten bei den Regensiebböden das aufsteigende Gas und die in der Trennkolonne absteigende Flüssigkeit durch die gleichen Durchtrittsstellen des Bodens. Auch beim Regensiebboden bedarf es wie beim Zwangssiebboden einer minimalen Dampfbelastung, um eine angemessene Trennwirkung zu erzielen. Wird sie signifikant unterschritten, bewegen sich aufsteigendes Gas und absteigender Rücklauf im wesentlichen ohne Austausch aneinander vorbei und der Boden läuft Gefahr, trocken zu laufen.

D.h., auch beim Regensiebboden muss eine untere Grenzgeschwindigkeit vorhanden sein, damit auf dem Boden eine gewisse Flüssigkeitsschicht gehalten wird, um ein Arbeiten des Bodens zu ermöglichen. Im normalen Arbeitsbereich regnet die Flüssigkeit bei Regensiebböden durch die Durchtrittsöffnungen von Boden zu Boden und zwischen den Böden wird die geschlossene Gasphase von einer zerteilten Flüssigkeitsphase durchsetzt. Erfindungsgemäß bevorzugt sind die vorgenannten Siebböden der einzusetzenden Rektifikationskolonne Regensiebböden.

Für das erfindungsgemäße Verfahren bevorzugte Siebboden-Rektifikationskolonnen sind die in der DE-A 10230219 beschriebenen. Ebenso kommen für das erfindungsgemäße Verfahren aber auch diejenigen Siebboden-Rektifikationskolonnen in Betracht, die in der DE-A 10156988 bzw. in der EP-A 1029573 beschrieben sind.

Hydraulisch abgedichtete Querstromböden sind gegenüber Siebböden dadurch charakterisiert, dass sie beim Abschalten der Kolonne nicht leer laufen können, sieht man von der winzigen Leerlaufbohrung (ihr Querschnitt ist normalerweise mehr als 200 mal kleiner als der Gesamtquerschnitt der Durchtrittsstellen) ab, die jeder Querstromboden aus Zweckmäßigkeitsgründen aufweist.

D.h., auch bei geringen Kolonnenbelastungen weisen hydraulisch abgedichtete Querstromböden gestaute Flüssigkeit (Rücklauf- und/oder Zulaufflüssigkeit) auf und laufen keine Gefahr, trocken zu laufen. Dies ist dadurch bedingt, dass es sich bei den Durchtrittstellen von hydraulisch abgedichteten Querstromböden nicht wie bei Siebböden um kaminlose Bohrungen handelt. Vielmehr mündet jede Durchtrittstelle in einen Kamin, der ein Trockenlaufen unterbindet. Über dem Kamin sind Dampfumlenkhauben (Glocken) angebracht, die in die gestaute Bodenflüssigkeit eintauchen. Häufig sind die Dampfumlenkhauben an ihren Rändern geschlitzt oder gezackt (d.h., sie weisen Treibschlitze auf). Der durch die Durchtrittsstelle aufsteigende Dampfstrom erfährt durch die Dampfumlenkhauben eine Ablenkung und strömt parallel zum Boden, d.h., quer zur Kolonne, in die gestaute Flüssigkeit.

Die aus benachbarten, in der Regel über dem Boden äquidistant verteilt angeordneten, Hauben austretenden Dampfblasen bilden in der gestauten Flüssigkeit eine Sprudelschicht aus.

Ablaufrohre bzw. -segmente, die, in der Regel abwechselnd links oder rechts, Böden verlassen, regeln - von Wehren unterstützt - den Flüssigkeitsstand der Stoffaustauschböden und führen die Flüssigkeit dem darunterliegenden Boden zu. Für die hydraulisch abdichtende Wirkung ist wesentlich, dass die Ablaufrohre bzw. -Segmente des oberen Bodens in die gestaute Flüssigkeit des darunter liegenden Bodens tauchen. Vorzugsweise sind keine Zulaufwehre vorhanden. In der Höhe einstellbare Glocken gestatten ein Anpassen an die Strömungsverhältnisse und den Ausgleich der Eintauchtiefen bei Herstellungsungleichmäßigkeiten, so dass alle Glocken des Bodens gleichmäßig gasen.

Je nach Gestalt und Anordnung der Glocken unterscheidet man z.B. die einflutig gestalteten hydraulisch abgedichteten Querstromböden in Rundglockenböden (Durchtrittstelle, Kamin und Glocke sind rund), Tunnel-Böden (Durchtrittstelle, Kamin und Glocke sind rechteckig, die Glocken sind hintereinander angeordnet, wobei die längere Rechteckkante parallel zur Querstromrichtung der Flüssigkeit ausgerichtet ist) und Thormann-Böden (Durchtrittstelle, Kamin und Glocke sind rechteckig, die Glocken sind hintereinander angeordnet, wobei die längere Rechteckkante senkrecht zur Querstromrichtung der Flüssigkeit ausgerichtet ist).

Unter Ventilböden sollen in dieser Schrift Querstromböden verstanden werden, die Bodenbohrungen mit hubbegrenzten Teller-, Ballast - oder Hebeventilen (Schwimmklappen) aufweisen, die die Größe der Dampfdurchtrittsöffnung der jeweiligen Kolonnenbelastung anpassen. Der aufsteigende Gasstrom wird abgelenkt, strömt parallel zum Boden in die gestaute Rücklaufflüssigkeit und bildet eine Sprudelschicht aus. Bewehrte Ablaufrohre führen den Rücklauf von Boden zu Boden. Häufig sind sie zweiflutig gestaltet. Sie können aber auch drei - und mehrflutig (z.B. bis zu achtflutig) gestaltet sein.

Selbstverständlich ist das erfindungsgemäße Verfahren auch dann anwendbar, wenn die Rektifikationskolonne eine Boden-Rektifikationskolonne ist, deren Stoffaustauschböden zu wenigstens 80 % ihrer Anzahl hydraulisch abgedichtete Querstromböden sind. Natürlich kann es sich auch bei der Gesamtzahl der Stoffaustauschböden um hydraulisch abgedichtete Querstromböden handeln. Häufig werden jedoch für das erfindungsgemäße Verfahren Bodenrektifikationskolonnen eingesetzt, deren Böden im oberen Teil der Rektifikationskolonne Ventilböden bilden.

Als hydraulisch abgedichtete Querstromböden werden erfindungsgemäß bevorzugt Thormann-Böden eingesetzt. Dies insbesondere dann, wenn im oberen Teil der Rektifikationskolonne Ventilböden eingesetzt werden.

Die für das erfindungsgemäße Verfahren erforderliche Wärmezufuhr erfolgt in zweckmäßiger Weise z.B. über innen- und/oder außenliegende Wärmetauscher herkömmlicher Bauart und/oder mittels Doppelwandheizung. Häufig werden außenliegende Umlaufverdampfer mit Natur- oder Zwangsumlauf eingesetzt.

Der Einsatz mehrerer in Reihe oder parallel geschalteter Verdampfer ist erfindungsgemäß möglich.

Die Zufuhr der thermischen Energie erfolgt beim erfindungsgemäßen Verfahren vorzugsweise mittels eines außenliegenden Zwangsumlaufverdampfers und besonders bevorzugt mittels eines außenliegenden Zwangsumlaufentspannungsverdampfers, wie er z.B. in der DE-A 10332758 und in der EP-A 854129 beschrieben ist.

Der Zwangsumlaufentspannungsverdampfer ist im Unterschied zum Zwangsumlaufverdampfer von der Rektifikationskolonne durch eine Drosselvorrichtung getrennt. Dem bei einem Druck Pₓ befindlichen flüssigen Inhalt der Trennkolonne wird kontinuierlich ein Teil entnommen und mittels einer Umlaufpumpe in die Zuströme eines z.B. Röhrenverdampfers (Rohrbündelwärmeaustauschers) gepumpt. Um die innenliegenden Rohre des Röhrenverdampfers strömt ein Wärmeträger, z.B. Heizdampf (in der Regel unter Druck stehender Wasserdampf), dessen Temperatur oberhalb der Temperatur des Flüssiginhalts der Trennkolonne liegt. Auf dem Weg durch die Zu- und Ausströmrohre des Röhrenverdampfers wird die entnommene Trennkolonnenflüssigkeit durch indirekten Wärmeaustausch auf eine Temperatur T_{y'} erhitzt, die oberhalb des Temperatur der Flüssiginhalts der Trennkolonne liegt.

Eine Drosselvorrichtung trennt Röhrenverdampfer und Trennkolonne druckseitig und ermöglicht durch geeignete Wahl der Umlaufpumpenleistung die Einstellung eines oberhalb von Pₓ gelegenen Drosselvordrucks P_{y'}, der oberhalb des zur Temperatur T_{y'} gehörigen Siededrucks P_{y'} der entnommenen Trennkolonnenflüssigkeit liegt. Durch vorstehende Maßnahmen wird ein Sieden des umgepumpten Trennkolonnenflüssigkeitsanteils in den Röhren des Röhrenverdampfers unterdrückt. Der umgepumpte Anteil der Trennkolonnenflüssigkeit wird in den Rohren des Röhrenverdampfers bezüglich des über dem flüssigen Inhalt der Trennkolonne herrschenden Drucks Pₓ vielmehr überhitzt und der Siedeprozess so auf die Durchtrittseite der Drosselvorrichtung verlagert (d.h., der Inhalt der Röhren des Röhrenverdampfers liegt einphasig vor, der Röhrenverdampfer fungiert lediglich als Überhitzer). Der Durchtritt der überhitzten Flüssigkeit durch die Drosselvorrichtung in die Trennkolonne kann dabei unmittelbar in den flüssigen Inhalt der Trennkolonne (des Trennkolonnensumpfes) hinein erfolgen. Unter diesen Bedingungen entspricht die Temperatur des flüssigen Inhalts des Trennkolonnensumpfes regelmäßig der zum über der Sumpfflüssigkeit herrschenden Druck Pₓ gehörigen Siedetemperatur Tₓ.

Prinzipiell kann der Durchtritt der überhitzten Flüssigkeit durch die Drosselvorrichtung in die Trennkolonne aber auch oberhalb des Flüssigkeitsspiegels des Trennkolonnensumpfes erfolgen. Unter diesen Bedingungen liegt die Temperatur des flüssigen Inhalts des Trennkolonnensumpfes regelmäßig unterhalb der zum über der Sumpfflüssigkeit herrschenden Druck Pₓ gehörigen Siedetemperatur Tₓ. Wesentlich ist, dass die Siedeverdampfungswirkung des außerhalb der Trennkolonne angebrachten z.B. Röhrenverdampfers erst in der Trennkolonne, d.h., außerhalb des Umlaufverdampfers, eintritt. Die Drosselung kann z.B. mechanisch (Blenden, Ventile) und/oder hydrostatisch (durch eine entsprechend hohe Sumpfsäule über der Durchtrittstelle der überhitzten Flüssigkeit) erfolgen.

Die Zufuhr der Flüssigkeit F in die Rektifikationskolonne kann beim erfindungsgemäßen Verfahren sowohl in den Sumpf der Rektifikationskolonne als auch in ihren Unterteil oder in ihren Mittelteil hinein erfolgen. Die Mitte wird dabei über die Anzahl der theoretischen Trennstufe der Rektifikationskolonne definiert. Dort wo sich (von unten nach oben betrachtet) die Hälfte der theoretischen Trennstufen befindet, ist die Mitte. Der Mittelteil befindet sich unmittelbar darunter und der Unterteil beginnt dort, wo der Mittelteil endet, nämlich unterhalb der Stelle der Rektifikationskolonne, wo das erste Drittel der theoretischen Trennstufen endet (von unten nach oben betrachtet).

Vorzugsweise wird das erfindungsgemäße Verfahren bei reduziertem Druck durchgeführt. Erfindungsgemäß besonders geeignete Kopfdrucke betragen ≤ 500 mbar, und besonders bevorzugt 10 bis 500 mbar, häufig 10 bis 200 mbar und vorzugsweise 10 bis 150 mbar. Der Druckverlust über die Rektifikationskolonne beträgt beim erfindungsgemäßen Verfahren vorteilhaft 300 bis 100, oder 250 bis 150 mbar. Die Temperatur im Sumpf der Rektifikationskolonne beträgt beim erfindungsgemäßen Verfahren zweckmäßig 100 bis 230°C und vorzugsweise 120 bis 210°C, bzw. 160 bis 200°C.

Selbstverständlich können in der erfindungsgemäß zu verwendenden Rektifikationskolonne auch Packungen und/oder Füllkörperschüttungen als trennwirksame Einbauten enthalten sein. Als Füllkörperschüttungen können z.B. Ringe, Wendeln, Sattelkörper, Raschig-, Intas- oder Pall-Ringe, Barrel- oder Intalox-Sättel, Top-Pak oder Geflechte verwendet werden. Natürlich können auch alle in dieser Schrift genannten möglichen Kolonneneinbauten in gemischter Form in der Rektifikationskolonne enthalten sein.

Wie bereits erwähnt, werden die sonstigen Polymerisationsinhibitoren beim erfindungsgemäßen Verfahren üblicherweise am Kopf der Kolonne aufgegeben. Sie können zusätzlich aber auch dem Sumpf zugefügt werden, und auch bereits in der aufzutrennenden, Acrylsäure enthaltenden, Flüssigkeit zugesetzt sein. Als typische Vertreter solcher Polymerisationsinhibitoren seien nochmals Phenothiazin, 4-Methoxyphenol und 4-Hydroxy-2,2,6,6-tetramethylpiperidin-N-oxyl genannt. Bezogen auf den Gehalt der Flüssigphase an Acrylsäure werden in der Regel bis zu wenigen hundert Gew.-ppm an Polymerisationsinhibitoren eingesetzt.

Es ist auch bekannt (vgl. z.B. DE-A 10256147, DE-A 19501325 und Chem. Eng. Technol. 21 (1998) 10, S. 829 bis 836) und erfindungsgemäß gleichfalls anwendbar, zum Zweck einer zusätzlichen Polymerisationsinhibierung die Rektifikationskolonne während der Rektifikation mit einem molekularen Sauerstoff enthaltenden Gas (z.B. Luft oder Magerluft) zu durchströmen (vgl. auch DE-A 10238145).

Die Entnahmestelle E für den Stoffstrom S kann sich beim erfindungsgemäßen Verfahren sowohl am Kolonnenkopf als auch als Seitenentnahme unterhalb des Kolonnenkopfes (z.B. im oberen Teil oder im Mittelteil der Rektifikationskolonne) befinden. Letzteres ist erfindungsgemäß bevorzugt.

Vorteilhaft befindet sich die Seitenentnahme wenigstens 0,2 bis 5 theoretische Trennstufen, häufig 0,5 bis 3 oder 0,6 bis 2 theoretische Trennstufen unterhalb des Kolonnenkopfes.

Die erfindungsgemäß aufzutrennende, Acrylsäure enthaltende Flüssigkeit kann die Acrylsäure sowohl in mehr oder weniger reiner Form als auch in Lösung befindlich enthalten.

Das Lösungsmittel kann dabei sowohl wässrig als auch ein organisches Lösungsmittel sein. Die spezifische Art des Lösungsmittels ist erfindungsgemäß weitgehend unbeachtlich. Vorzugsweise ist es wässrig oder ein organisches Lösungsmittel mit gegebenenfalls geringfügigen wässrigen Anteilen.

Der Gehalt an Acrylsäure in der Flüssigkeit F kann ≥ 2 Gew.-%, oder ≥ 5 Gew.-%, oder ≥ 10 Gew.-%, oder ≥ 20 Gew.-%, oder ≥ 40 Gew.-%, oder ≥ 60 Gew.-%, oder ≥ 80 Gew.-%, oder ≥ 90 Gew.-%, oder ≥ 95 Gew.-%, oder ≥ 99 Gew.-% betragen.

Insbesondere lässt sich das erfindungsgemäße Verfahren dann anwenden, wenn das rektifikative Verfahren die Abtrennung von Acrylsäure aus einem Acrylsäure (in der Regel 10 bis 35 Gew.-%, häufig 15 bis 30 Gew.-% bezogen auf das Gewicht der Flüssigkeit) und eine höher als Acrylsäure siedende (vorzugsweise inerte) hydrophobe organische Flüssigkeit als Hauptbestandteile sowie (z.B. niedere) Aldehyde als Nebenbestandteile (z.B. bis zu 10000 oder 5000 Gew.-ppm) enthaltenden Gemisch betrifft (dabei kann der Flüssigkeit F vorab ihrer erfindungsgemäßen rektifikativen Behandlung ein primäres Amin und/oder dessen Salz zugesetzt werden, wie es z.B. die EP-A 717029 beschreibt), wie es z. B. im Rahmen der Abtrennung von Acrylsäure aus dem Reaktionsgasgemisch der katalytischen Gasphasenoxidation gemäß den in der DE-A 44 36 243, der DE-PS 21 36 396, der EP-A 925 272 und der DE-A 43 08 087 bzw. DE-A 10336386 beschriebenen Verfahrensweisen auftritt (der entnommene Stoffstrom S enthält dabei meist nicht mehr als 99,5 Gew.%, häufig nicht mehr als 99 Gew.% und vielfach nicht mehr als 98,5 Gew.-% Acrylsäure). D.h., wenn das Acrylsäure und eine höher als Acrylsäure siedende inerte hydrophobe organische Flüssigkeit als Hauptbestandteile sowie niedere Aldehyde als Nebenbestandteile enthaltende Einsatzgemisch für das erfindungsgemäße Verfahren z.B. aus den Reaktionsgasgemischen der katalytischen Gasphasenoxidation als Flüssigkeitsablauf einer Gegenstromabsorption mit anschließender Desorption durch Abstreifen gemäß der DE-PS 21 36 39, der EP-A 925 272 oder der DE-A 43 08 087 bzw. DE-A 10336386 oder als Flüssigkeitsablauf einer Gegenstromabsorption mit überlagerter Rektifikation gemäß der DE-A 44 36 243 erhalten wurde. Unter hochsiedender inerter hydrophober organischer Flüssigkeit sind hier solche Flüssigkeiten zu verstehen, deren Siedepunkt bei Normaldruck (1 atm) oberhalb der Siedetemperatur der Acrylsäure liegt und in denen die Löslichkeit (Gew.%, bezogen auf das Gewicht der Lösung) von Acrylsäureoligo- und/oder -polymerisaten bei 25°C und 1 atm geringer als in reiner Acrylsäure ist. Insbesondere sind es solche hochsiedenden organischen Flüssigkeiten, die zu wenigstens 70 Gew-% aus solchen Molekülen bestehen, die keine nach außen wirkende polare Gruppe enthalten und somit beispielsweise nicht in der Lage sind, Wasserstoffbrücken zu bilden. Im engeren Sinn umfasst der Begriff hier die hochsiedenden organischen Absorptionsflüssigkeiten, die in der DE-PS 21 36 396, der DE A43 08 087 sowie der DE-A 44 36 243 empfohlen werden.

Dies sind im wesentlichen Flüssigkeiten, deren Siedepunkt bei Normaldruck oberhalb von 160°C liegt. Beispielhaft genannt seien Mittelölfraktionen aus der Paraffindestillation, Diphenylether, Diphenyl oder Mischungen der vorgenannten Flüssigkeiten wie z.B. ein Gemisch aus 70 bis 75 Gew.-% Diphenylether und 25 bis 30 Gew.-% Diphenyl (Diphyl genannt). Eine günstige hochsiedende hydrophobe organische Absorptionsflüssigkeit ist auch ein Gemisch, bestehend aus einer Mischung aus 70 bis 75 Gew.-% Diphenylether und 25 bis 30 Gew.-% Diphenyl, sowie, bezogen auf diese Mischung, 0,1 bis 42,5 Gew.-% o-Dimethylphthalat.

D.h., das erfindungsgemäße Verfahren eignet sich u.a. für Flüssigkeiten F, die nachfolgende Komponenten enthalten (die Mengenanteile sind auf die Gesamtmenge bezogen):

| | |
|---|---|
| Diphyl | 50 bis 75 Gew.-%, |
| Dimethylphthalat | 10 bis 25 Gew.-%, |
| Diacrylsäure | 0,2 bis 3 Gew.-%, |
| Acrylsäure | 15 bis 35 Gew.-%, häufig 15 bis 25 Gew.-%, |
| Wasser | 0,07 bis 0,2 Gew.-%, |
| Essigsäure | 0,01 bis 0,2 Gew.-%, |
| Ameisensäure | 0,001 bis 0,02 Gew.-%, |
| Propionsäure | 0,001 bis 0,02 Gew.%, |
| Phenothiazin | 0,01 bis 0,1 Gew.-%, |
| Phthalsäureanhydrid | 0,1 bis 1 Gew.-%, |
| Benzoesäure | 0,2 bis 2 Gew.-%, |
| Maleinsäureanhydrid | 0,1 bis 2 Gew.-%, |
| Benzaldehyd | 0,1 bis 1 Gew.-% und |
| Furfurale | 0,01 bis 0,05 Gew.-%. |

Die dabei zu verwendende Rektifikationseinheit kann von an sich bekannter Bauart sein und die üblichen Einbauten aufweisen. Als Kolonneneinbauten kommen prinzipiell alle gängigen Einbauten in Betracht, beispielsweise Böden, Packungen und/oder Schüttungen. Unter den Böden sind Glockenböden, Siebböden, Ventilböden, Thormannböden und/oder Dual-Flow-Böden bevorzugt, unter den Schüttungen sind solche mit Ringen, Wendeln, Sattelkörpern, Raschig-, Intos- oder Pall-Ringen, Barrel- oder Intalox-Sätteln, Top-Pak etc. oder Geflechten bevorzugt. Besonders bevorzugt sind Dual-Flow-Böden.

In der Regel sind 10 bis 25 theoretische Böden in der Rektifikationseinheit ausreichend. Die Rektifikation wird üblicherweise bei vermindertem Druck durchgeführt, vorzugsweise bei einem Kopfdruck von 70 bis 140 mbar. Der Sumpfdruck ergibtsich aus dem Kopfdruck, der Anzahl und der Art der Kolonneneinbauten sowie den fluidynamischen Erfordernissen der Rektifikation und beträgt vorzugsweise 300 bis 400 mbar.

Bei Verwendung von Dual-Flow-Böden als trennwirksamen Einbauten beträgt der Lochdurchmesser in den Böden oberhalb des Zulaufs in der Regel 5 bis 25 mm, vorzugsweise 10 bis 20 mm. Der Lochdurchmesser in den Böden unterhalb des Zulaufs beträgt üblicherweise 15 bis 80 mm, vorzugsweise 25 bis 50 mm und ist ganz besonders bevorzugt gestuft, wie in der deutschen Patentanmeldung DE - A 10156988 beschrieben. Der Bodenabstand (in der Regel sind die Böden äquidistant angeordnet) beträgt typisch 300 bis 700 mm, bevorzugt 350 bis 400 mm und ganz besonders bevorzugt 380 mm.

Bevorzugt wird der obere Teil der Rektifikationseinheit begleitbeheizt. Bei einer Rektifikationseinheit mit n theoretischen Böden betrifft dies den Bereich oberhalb des n/2 ten theoretischen Bodens. Die Temperatur der Begleitheizung wird so gewählt, dass an der Kolonneninnenwand keine Acrylsäure kondensieren kann. Vorzugsweise liegt diese Temperatur 5 bis 10°C oberhalb der Siedetemperatur von Acrylsäure bei dem in der Rektifikationseinheit im betrachteten Bereich vorliegenden Druck.

Die Rektifikationseinheit ist üblicherweise aus austenitischem Stahl gefertigt, vorzugweise aus dem Werkstoff 1.4571 (nach DIN EN 10020).

Der Zulauf in die Rektifikationseinheit erfolgt zweckmäßig in ihrem unteren Bereich. Vorzugsweise erfolgt er 2 bis 5 theoretische Böden (Trennstufen) oberhalb des Sumpfes der Rektifikationskolonne. Die Zulauftemperatur beträgt typisch 150°C.

Die Wärmezufuhr erfolgt über innen- und/oder außenliegende Wärmetauscher (Wärmeträger ist wieder vorzugsweise Wasserdampf) herkömmlicher Bauart und/oder über Doppelwandbeheizung. Vorzugsweise erfolgt sie über außenliegende Umlaufverdampfer mit Natur- oder Zwangsumlauf. Besonders bevorzugt sind außenliegende Umlaufverdampfer mit Zwangsumlauf. Der Einsatz mehrerer Verdampfer, in Reihe oder parallel geschaltet , ist möglich. Vorzugsweise werden 2 bis 4 Verdampfer parallel betrieben. Die Sumpftemperatur der Rektifikationseinheit beträgt typisch 170 bis 210°C, vorzugsweise 180 bis 200°C. Die in den Sumpf der Rektifikationskolonne kondensierende Schwersiederfraktion enthält in der Regel im wesentlichen folgende Bestandteile (die Mengenanteile sind auf die Gesamtmenge bezogen):

| | |
|---|---|
| Diphyl | 70 - 85 Gew.-%, |
| Dimethylphthalat | 10 - 25 Gew.%, |
| Diacrylsäure | 0,5 - 5 Gew.-%, |
| Acrylsäure | 0,2 - 2 Gew.-%, |
| Phenothiazin | 0,01 - 0,1 Gew.-%, |
| Phthalsäureanhydrid | 0,1 - 1 Gew.%, |
| Benzoesäure | 0,2 - 2 Gew.-%, |
| Maleinsäureanhydrid | 0,1-2 Gew.-%, |
| Benzaldehyd | 0,1 - 0,5 Gew.-% und |
| Furfurale | 0,01 - 0,05 Gew.-%. |

Die der Rektifikationseinheit entnommene, das schwersiedende Absorptionsmittel einkondensiert enthaltende, Sumpfflüssigkeit wird teilweise ausgeschleust, vorzugsweise 75 bis 90 Gew.-% bezogen auf den Zulauf in die Rektifikationseinheit, und teilweise über einen Wärmetauscher in den Sumpfbereich der Rektifikationskolonne zurückgeführt.

Oberhalb des Zulaufs in die Rektifikationskolonne wird über Seitenabzug eine rohe Acrylsäure entnommen, vorzugsweise 8 bis 20 theoretische Böden oberhalb des Kolonnensumpfes. Die Entnahme der rohen Acrylsäure erfolgt auf übliche Weise und unterliegt keiner Beschränkung. Geeignet ist die Entnahme über einen Fangboden, wobei der gesamte Rücklauf aufgefangen wird und ein Teil ausgeschleust und der andere Teil als Rücklauf unterhalb des Fangbodens verwendet wird, oder über einen Boden mit integrierter Abzugsmöglichkeit, vorzugsweise über einen Dual-Flow-Boden mit integrierter Abzugsmöglichkeit. Die entnommene rohe Acrylsäure enthält normalerweise im wesentlichen folgende Bestandteile (die Mengenanteile sind auf die Gesamtmenge bezogen):

| | |
|---|---|
| Acrylsäure | 98 - 99,9 Gew.%, |
| Essigsäure | 0,05 - 0,3 Gew.%, |
| Wasser | 0,001 - 0,05 Gew.-%, |
| Ameisensäure | 0,001 -0,005 Gew.-%, |
| Propionsäure | 0,01 - 0,05 Gew.-%, |
| Furfurale | 0,01 - 0,05 Gew.-%, |
| Allyacrylat | 0,001 - 0,01 Gew.-%, |
| Benzaldehyd | 0,001 - 0,01 Gew.-%, |
| Maleinsäureanhydrid | 0,002 -0,02 Gew.-%, |
| Diacrylsäure | 0,01 - 0,05 Gew.-% und |
| Phenothiazin | 0,01 - 0,05 Gew.-%. |

Die entnommene rohe Acrylsäure wird mittels kalter roher (vorab gewonnener) Acrylsäure und/oder mittels eines Wärmetauschers abgekühlt (als Kühlmittel eignen sich z.B. Oberflächenwässer). Der Einsatz mehrerer Wärmetauscher, in Reihe oder parallel geschaltet, ist möglich. In den Wärmetauschern, die dem Fachmann an sich bekannt sind und keiner besonderen Beschränkung unterliegen, wird die rohe Acrylsäure vorzugsweise um 40 bis 70°C abgekühlt.

Die entnommene rohe Acrylsäure, vorzugsweise 10 bis 25 Gew.-% bezogen auf den Zulauf in die Rektifikationskolonne, wird ausgeschleust und teilweise als Lösemittel für den Polymerisationsinhibitor verwendet.

Die Abkühlung des am Kopf der Rektifikationskolonne abgetrennten Leichtsiederstroms kann indirekt, beispielsweise durch Wärmetauscher (als Kühlmittel können z.B. Oberflächenwässer verwendet werden), die dem Fachmann an sich bekannt sind und keiner besonderen Beschränkung unterliegen, oder direkt, beispielsweise durch einen Quench, erfolgen. Vorzugsweise erfolgt sie durch direkte Kühlung. Dazu wird bereits kondensierte Leichtsiederfraktion mittels eines geeigneten Wärmetauschers gekühlt und die gekühlte Flüssigkeit oberhalb der Abnahmestelle im Brüden versprüht. Dieses Versprühen kann in einem getrennten Apparat oder in der Rektifikationseinheit selbst erfolgen. Beim Versprühen in der Rektifikationseinheit ist die Entnahmestelle der Leichtsiederfraktion vorteilhafterweise als Fangboden ausgebildet. Durch Einbauten, die die Durchmischung der gekühlten Leichtsiederfraktion mit dem Brüden verbessern, kann die Wirkung der direkten Kühlung gesteigert werden. Hierzu kommen prinzipiell alle gängigen Einbauten in Betracht, beispielsweise Böden, Packungen und/oder Schüttungen. Unter den Böden sind Glockenböden, Siebböden, Ventilböden, Thormannböden und/oder Dual-Flow-Böden bevorzugt. Unter den Schüttungen sind solche mit Ringen, Wendeln, Sattelkörpern, Raschig-, Intos- oder Pall-Ringen, Barrel- oder Intalox-Sätteln, Top-Pak etc. oder Geflechten bevorzugt. Besonders bevorzugt sind Dual-Flow-Böden. In der Regel sind hier 2 bis 5 theoretische Böden ausreichend. Diese Böden sind bei den bisher erfolgten Angaben zur Anzahl der theoretischen Böden der Rektifikationseinheit nicht berücksichtigt. Die direkte Kondensation der Leichtsiederfraktion kann auch mehrstufig ausgeführt sein, mit nach oben abnehmender Temperatur. Der am Kopf der Rektifikationskolonne abgetrennte Leichtsiederstrom enthält im wesentlichen folgende Komponenten (die Mengenanteile sind auf die Gesamtmenge bezogen):

| | |
|---|---|
| Acrylsäure | 90 - 99 Gew.-%, |
| Essigsäure | 0,5 - 3 Gew.-%, |
| Wasser | 0,5 - 2 Gew.-%, |
| Phenothiazin | 0,02 - 0,1 Gew.-%, |
| Allylacrylat | 0,01 - 0,1 Gew.-%, |
| Furfurale | 0,002 - 0,01 Gew.-%, |
| Propionsäure | 0,01 - 0,05 Gew.-%, |
| Ameisensäure | 0,1 - 1 Gew.-% und |
| Acrolein | 0,001 - 0,002 Gew.-%. |

Ein Teil der als Leichtsiederfraktion entnommenen Flüssigkeit, vorzugsweise 30 bis 50 Gew.-% bezogen auf den Zulauf in die Rektifikationskolonne, wird als Rücklauf verwendet, der Rest der Leichtsiederfraktion, vorzugsweise 0,5 bis 2 Gew.-% bezogen auf den Zulauf in die Rektifikationskolonne, wird ausgeschleust. In erfindungsgemäß vorteilhafter Weise wird die beschriebene Rektifikation nun so betrieben, dass der Diacrylsäuregehalt im Rücklauf, bezogen auf dessen Gewicht, wenigstens 550 Gew.-ppm beträgt. Bevorzugt wird der vorgenannte Diacrylsäuregehalt 1000 Gew.-ppm oder sogar 1500 Gew.-ppm oder mehr betragen.

Das Abgas aus der Rektifikationseinheit enthält (wenn die im folgenden noch auszuführende Sauerstoff- bzw. Luftmitinhibierung angewendet wird) in der Regel im wesentlichen folgende Bestandteile(die Mengenanteile sind auf die Gesamtmenge bezogen):

| | |
|---|---|
| Stickstoff | 70 - 80 Gew.-%, |
| Sauerstoff | 15 - 25 Gew.%, häufig 15 bis 21 Gew.-%, |
| Acrylsäure | 1-5 Gew.-%, |
| Essigsäure | 0,05 - 0,5 Gew.-%, |
| Wasser | 0,2 - 0,8 Gew.-%, |
| Acrolein | 0,001 0,005 Gew.-%, |
| Ameisensäure | 0,02 - 0,2 Gew.-%, |
| Allylacrylat | 0,001 - 0,005 Gew.-% und |
| Kohlenoxide | 0,02 - 0,06 Gew.-%. |

Das Abgas wird zusammen mit anderen Produktionsrückständen verbrannt.

Als Polymerisationsinhibitor können in der Rektifikationskolonne Alkylphenole, beispielsweise o-, m- oder p-Kresol (Methylphenol), 2-tert.-Butyl-4-methylphenol, 6-tert.-Butyl-2,4-dimethyl-phenol, 2,6-Di-tert.-Butyl-4-methylphenol, 2-tert.-Butylphenol, 4-tert.-Butylphenol, 2,4-di-tert.-Butylphenol, 2-Methyl-4-tert.-Butylphenol, 4-tert.-Butyl-2,6-dimethylphenol, oder 2,2'-Methylen-bis-(6-tert.-butyl-4-methylphenol), Hydroxyphenole, beispielsweise Hydrochinon, 2-Methylhydrochinon, 2,5-Di-tert.-Butylhydrochinon, Brenzcatechin (1,2-Dihydroxybenzol) oder Benzochinon, Aminophenole, wie z.B. paraAminophenol, Nitrosophenole, wie z.B. para-Nitrosophenol, Alkoxyphenole, beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Di- tert.-Butyl-4-methoxyphenol, Tocopherole, wie z.B. α-Tocopherol sowie 2,3-Dihydro-2,2-dimethyl-7-hydroxybenzofuran (2,2-Dimethyl-7-hydroxycumaran), N-Oxyle, wie 4-Hydroxy-2,2,6,6-tetramethyl-piperidin-N-oxyl, 4-Oxo-2,2,6,6-tetramethyl-piperidin-N-oxyl, 4-Acetoxy-2,2,6,6-tetramethyl-piperidin-N-oxyl, 2,2,6,6-Tetramethyl-piperidin-N-oxyl, 4,4',4"-Tris(2,2,6,6-tetramethyl-piperidin-N-oxyl)-phosphit oder 3-Oxo-2,2,5,5-tetramethyl-pyrrolidin-N-oxyl, aromatische Amine oder Phenylendiamine, wie z.B. N,N-Diphenylamin, N-Nitroso-diphenylamin, N,N'-Dialkyl-para-phenylendiamin, wobei die Alkylreste gleich oder verschieden sein können und jeweils unabhängig voneinander aus 1 bis 4 Kohlenstoffatome bestehen und geradkettig oder verzweigt sein können, Hydroxylamine, wie z.B. N,N-Diethylhydroxylamin, phosphorhaltige Verbindungen, wie z.B. Triphenylphosphin, Triphenylphosphit, hypophosphorige Säure oder Triethylphosphit, schwefelhaltige Verbindungen, wie z.B. Diphenylsulfid oder Phenothiazin, gegebenenfalls in Kombination mit Metallsalzen, wie beispielsweise die Chloride, Dithiocarbamate, Sulfate, Salicylate oder Acetate von Kupfer, Mangan, Cer, Nickel oder Chrom verwendet werden. Selbstverständlich können auch Gemische der genannten Polymerisationsinhibitoren eingesetzt werden.

Bevorzugt wird in der Rektifikationseinheit Phenothiazin als Polymerisationsinhibitor verwendet oder ein anderer Polymerisationsinhibitor, der die gleiche Wirksamkeit wie Phenothiazin zeigt.

Der Polymerisationsinhibitor wird vorzugsweise als Lösung in relativ reiner Acrylsäure, besonders bevorzugt als Lösung in der über Seitenabzug abgetrennten rohen Acrylsäure, dem kondensierten Leichtsiederrücklauf zugesetzt. Die Gesamtkonzentration an aldehydischen Verunreinigungen in der für den Stablilisatoransatz verwendeten Acrylsäure beträgt vorzugsweise weniger als 500 Gew.-ppm. Die Stabilisator(Inhibitor)-konzentration im Stabilisatoransatz (in der Stabilisatorlösung) hängt vom verwendeten Stabilisator und dem verwendeten Lösemittel ab. Die optimale Stabilisatorkonzentration im Stabilisatoransatz kann durch Löseversuche ermittelt werden und beträgt im Falle von Phenothiazin als Stabilisator und von roher Acrylsäure als Lösemittel 0,1 bis 2 Gew.-%, vorzugsweise 1,2 bis 1,7 Gew.-%. Der Stabilisator wird so dosiert, dass die Stabilisatorkonzentration in der ausgeschleusten rohen Acrylsäure 50 bis 500 Gew.-ppm, vorzugsweise 150 bis 350 Gew.-ppm, beträgt.

Die Form der festen Polymerisationsinhibitoren, die in den Stabilisatoransatzbehälter eingebracht werden, ist prinzipiell beliebig. Beispielsweise kommen Schuppen, Granulat oder Pellets in Betracht. Wegen des geringeren Staubanteils und des günstigeren Rieselverhaltens sind Pellets bevorzugt. Die festen Polymerisationsinhibitoren können beispielsweise mittels Förderschnecken aus einem Silo in den Stabilisatoransatzbehälter eingebracht werden.

Zur weiteren Unterstützung der Stabilisierung (Polymerisationsinhibierung) kann ein sauerstoffhaltiges Gas, bevorzugt Luft oder ein Gemisch aus Luft und Stickstoff (Magerluft), besonders bevorzugt Luft, anwesend sein.

Dieses sauerstoffhaltige Gas wird vorzugsweise in den Sumpfbereich der Rektifikationskolonne und/oder in einen Umlaufverdampfer eindosiert.

Die Luft wird zweckmäßig unmittelbar der Atmosphäre entnommen, vorzugsweise an einer oder mehreren Probenahmestellen.

Erfindungsgemäß bevorzugt wird die so mitverwendete Luft vorab ihrer Verwendung ebenfalls, wie bereits im Zusammenhang mit der Partialoxidation beschrieben, filtriert.

Ganz besonders bevorzugt wird Luft mit einem Salzgehalt, z.B. an organischen und anorganischen Chloriden, insbesondere an Alkalichloriden, von weniger als 10 Gew.-ppm verwendet.

Das sauerstoffhaltige Gas wird so dosiert, dass der Sauerstoffpartialdruck am Kopf der Rektifikationseinheit mindestens 0,5 mbar, vorzugsweise mindestens 2 mbar, beträgt.

Zusätzlich kann ein Tensid in die Rektifikationseinheit dosiert werden, wie es beispielsweise in der DE - A 19810962 beschrieben ist.

Das erfindungsgemäße Verfahren ist jedoch auch auf die rektifikative Weiterreinigung von Acrylsäurequalitäten anwendbar, die bereits 95 bis 99,5 oder 99,8 Gew.-% Acrylsäure enthalten (es ist insbesondere dann günstig, wenn diese ≥ 2, oder ≥ 5 Gew.ppm, oder ≥ 10 Gew.ppm, oder ≥ 20 Gew.ppm, oder ≥ 50 Gew.ppm an Aldehyden wie z.B. Acrolein, Furfurale, Benzaldehyd etc. enthalten; insbesondere dann, wenn deren Siedepunkt bei Normaldruck (1 atm) unterhalb des Siedepunktes von Acrylsäure liegt). Diese können vorab der Rektifikation mit Aminen wie Hydrazin oder Hydrazinderivaten (z.B. Aminoguanidinhydrogencarbonat) vorbehandelt worden sein, wie es z.B. in der DE-A 10219592 der EP-A 713854 und der EP-A 270999 beschrieben ist.

### Beispiele und Vergleichsbeispiel

Es wurden vier Proben von jeweils 0,5 ml frisch zubereitet (Destillation, dann einfrieren), deren Gehalt an Acrylsäure in allen Fällen 99,8 Gew.-% betrug.

Anschließend wurde der Diacrylsäuregehalt der vier Proben durch Zusatz von im wesentlichen reiner Diacrylsäure auf folgende Werte eingestellt:
- Probe 1:: ≤ 5 Gew.-ppm;
- Probe 2:: 500 Gew.-ppm;
- Probe 3:: 1000 Gew.-ppm;
- Probe 4:: 1500 Gew.-ppm;
- Probe 5:: 10000 Gew.-ppm;
- Probe 6:: 15000 Gew.-ppm.

Weiterer Polymerisationsinhibitor war in den Acrylsäureproben nicht enthalten. Zusätzlich wurde allen vier Proben soviel Benzaldehyd zugesetzt, dass sie einen Benzaldehydgehalt von 500 Gew.-ppm aufwiesen.

Unter Luftatmosphäre wurde jede der Proben in eine 1,8 ml Glasampulle überführt. Unmittelbar nach Fertigstellung wurden die Ampullen bei 120°C im Umlufttrockenschrank drehend gelagert, um vollständige Durchmischung zu gewährleisten. Dann wurde die Zeit T bis zur vollständigen Polymerisation der jeweiligen Probe visuell erfasst.

Die Versuchsreihe wurde dreimal wiederholt und der berichtete Wert arithmetisch gemittelt.

Die mittleren Ergebnisse für die Zeit T der jeweiligen Proben betrugen:
- Probe 1:: 128 min;
- Probe 2:: 136 min;
- Probe 3:: 143 min;
- Probe 4:: 153 min;
- Probe 5:: 175 min;
- Probe 6:: 195 min.

US Provisional Application Nr. 60/509,540 eingereicht am 9. Oktober 2003 ist eingefügt in die vorliegende Anmeldung durch Literaturhinweis.

Im Hinblick auf die obengenannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

## Patentansprüche

1. Ein Verfahren der rektifikativen Auftrennung einer Acrylsäure enthaltenden Flüssigkeit F, bei dem man die Acrylsäure enthaltende Flüssigkeit F über eine Zuführstelle Z einer Rektifikationskolonne zuführt und oberhalb der Zuführstelle Z der Rektifikationskolonne an einer Entnahmestelle E einen Stoffstrom S entnimmt, dessen Gehalt an Acrylsäure, bezogen auf das Gewicht des Stoffstroms S, ≥ 90 Gew.-% beträgt und größer ist als der entsprechende Acrylsäuregehalt der Flüssigkeit F in Gew.-%, **dadurch gekennzeichnet, dass** innerhalb des wenigstens zwei theoretische Trennstufen oberhalb der Zuführstelle Z befindlichen Bereichs der Rektifikationskolonne der Gehalt G der Rücklaufflüssigkeit an Diacrylsäure, bezogen auf das Gewicht der Rücklaufflüssigkeit, wenigstens in Teilbereichen ≥ 550 Gew.-ppm beträgt.

2. Verfahren nach Anspruch 1, **dadurch gekennzeichnet, dass** der Gehalt G der Rücklaufflüssigkeit an Diacrylsäure ≥ 650 Gew.-ppm beträgt.

3. Verfahren nach Anspruch 1, **dadurch gekennzeichnet, dass** der Gehalt G der Rücklaufflüssigkeit an Diacrylsäure ≥ 1000 Gew.-ppm beträgt.

4. Verfahren nach einem der Ansprüche 1 bis 3, **dadurch gekennzeichnet, dass** der Diacrylsäuregehalt G der Rücklaufflüssigkeit im gesamten wenigstens zwei theoretische Trennstufen oberhalb der Zuführstelle Z befindlichen Bereich der Rektifikationskolonne gegeben ist.

5. Verfahren nach einem der Ansprüche 1 bis 4, **dadurch gekennzeichnet, dass** der Diacrylsäuregehalt G der Rücklaufflüssigkeit im gesamten wenigstens vier theoretische Trennstufen oberhalb der Zuführstelle Z befindlichen Bereich der Rektifikationskolonne gegeben ist.

6. Verfahren nach einem der Ansprüche 1 bis 5, **dadurch gekennzeichnet, dass** sich die Entnahmestelle E wenigstens 2 theoretische Trennstufen oberhalb der Zuführstelle Z der Rektifikationskolonne befindet.

7. Verfahren nach einem der Ansprüche 1 bis 6, **dadurch gekennzeichnet, dass** die aufzutrennende Flüssigkeit F eine tiefer als Acrylsäure siedende Brönsted-Säure mit pKₛ ≤ 16 zugesetzt enthält.

8. Verfahren nach einem der Ansprüche 1 bis 7, **dadurch gekennzeichnet, dass** der Rücklaufflüssigkeit Diacrylsäure als solche und/oder Diacrylsäure enthaltende Acrylsäure zugesetzt wird.

9. Verfahren nach einem der Ansprüche 1 bis 8, **dadurch gekennzeichnet, dass** die Rektifikationskolonne wenigstens einen Siebboden als trennwirksame Einbaute enthält.

10. Verfahren nach einem der Ansprüche 1 bis 8, **dadurch gekennzeichnet, dass** die Rektifikationskolonne wenigstens einen hydraulisch abgedichteten Querstromboden als trennwirksame Einbaute enthält.

11. Verfahren nach einem der Ansprüche 1 bis 10, **dadurch gekennzeichnet, dass** für das erfindungsgemäße Verfahren erforderliche thermische Energie über einen außenliegenden Zwangsumlaufentspannungsverdampfer zugeführt wird.

12. Verfahren nach Anspruch 11, **dadurch gekennzeichnet, dass** der Zwangsumlaufentspannungsverdampfer ein Röhrenverdampfer ist.

13. Verfahren nach einem der Ansprüche 1 bis 12, **dadurch gekennzeichnet, dass** die Flüssigkeit F ein Gemisch ist, das 10 bis 35 Gew.-% Acrylsäure, 50 bis 80 Gew.% einer höher als Acrylsäure siedenden hydrophoben organischen Flüssigkeit und bis zu 5000 Gew.-ppm Aldehyde enthält.

14. Verfahren nach einem der Ansprüche 1 bis 13, **dadurch gekennzeichnet, dass** die Flüssigkeit F die nachfolgenden Komponenten enthält:
| | |
|---|---|
| Diphyl | 50 bis 75 Gew.-%, |
| Dimethylphthalat | 10 bis 25 Gew.-%, |
| Diacrylsäure | 0,2 bis 3 Gew.-%, |
| Acrylsäure | 15 bis 35 Gew.-%, häufig 15 bis 25 Gew.-%, |
| Wasser | 0,07 bis 0,2 Gew.-%, |
| Essigsäure | 0,01 bis 0,2 Gew.-%, |
| Ameisensäure | 0,001 bis 0,02 Gew.-%, |
| Propionsäure | 0,001 bis 0,02 Gew.-%, |
| Phenothiazin | 0,01 bis 0,1 Gew.-%, |
| Phthalsäureanhydrid | 0,1 bis 1 Gew.-%, |
| Benzoesäure | 0,2 bis 2 Gew.-%, |
| Maleinsäureanhydrid | 0,1 bis 2 Gew.-%, |
| Benzaldehyd | 0,1 bis 1 Gew.-% und |
| Furfurale | 0,01 bis 0,05 Gew.-%. |

15. Verfahren nach einem der Ansprüche 1 bis 12, **dadurch gekennzeichnet, dass** die Flüssigkeit F 95 bis 99,8 Gew.-% Acrylsäure enthält.

## Claims

1. A process for rectificatively separating an acrylic acid-comprising liquid F by feeding the acrylic acid-comprising liquid F to a rectification column via a feed point Z and withdrawing a stream S at a withdrawal point E above the feed point Z of the rectification column, said stream S having a content of acrylic acid, based on the weight of the stream S, which is ≥ 90% by weight and is greater than the corresponding acrylic acid content of the liquid F in % by weight, wherein the diacrylic acid content G of the reflux liquid within the region of the rectification column at least two theoretical plates above the feed point Z is at least in places ≥ 550 ppm by weight based on the weight of the reflux liquid.

2. The process according to claim 1, wherein the diacrylic acid content G of the reflux liquid is ≥ 650 ppm by weight.

3. The process according to claim 1, wherein the diacrylic acid content G of the reflux liquid is ≥ 1000 ppm by weight.

4. The process according to any of claims 1 to 3, wherein the diacrylic acid content G of the reflux liquid is satisfied within the entire region of the rectification column at least two theoretical plates above the feed point Z.

5. The process according to any of claims 1 to 4, wherein the diacrylic acid content G of the reflux liquid is satisfied within the entire region of the rectification column at least four theoretical plates above the feed point Z.

6. The process according to any of claims 1 to 5, wherein the withdrawal point E is at least two theoretical plates above the feed point Z of the rectification column.

7. The process according to any of claims 1 to 6, wherein the liquid F to be separated comprises an added Brønsted acid having a lower boiling point than acrylic acid and pKₐ ≤ 16.

8. The process according to any of claims 1 to 7, wherein diacrylic acid as such and/or diacrylic acid-comprising acrylic acid are added to the reflux liquid.

9. The process according to any of claims 1 to 8, wherein the rectification column comprises at least one sieve tray as a separating internal.

10. The process according to any of claims 1 to 8, wherein the rectification column comprises at least one hydraulically sealed crossflow tray as a separating internal.

11. The process according to any of claims 1 to 10, wherein the thermal energy required for the process according to the invention is supplied via an external forced-circulation flash evaporator.

12. The process according to claim 11, wherein the forced-circulation flash evaporator is a tubular evaporator.

13. The process according to any of claims 1 to 12, wherein the liquid F is a mixture which comprises from 10 to 35% by weight of acrylic acid, from 50 to 80% by weight of a hydrophobic organic liquid having a higher boiling point than acrylic acid and up to 5000 ppm by weight of aldehyde.

14. The process according to any of claims 1 to 13, wherein the liquid F comprises the following components:
Diphyl 50 to 75% by weight,
dimethyl phthalate 10 to 25% by weight,
diacrylic acid 0.2 to 3% by weight,
acrylic acid 15 to 35% by weight, frequently 15 to 25% by weight,
water 0.07 to 0.2% by weight,
acetic acid 0.01 to 0.2% by weight,
formic acid 0.001 to 0.02% by weight,
propionic acid 0.001 to 0.02% by weight,
phenothiazine 0.01 to 0.1% by weight,
phthalic anhydride 0.1 to 1% by weight,
benzoic acid 0.2 to 2% by weight,
maleic anhydride 0.1 to 2% by weight,
benzaldehyde 0.1 to 1% by weight and
furfurals 0.01 to 0.05% by weight.

15. The process according to any of claims 1 to 12, wherein the liquid F comprises from 95 to 99.8% by weight of acrylic acid.

## Revendications

1. Procédé de séparation rectificative d'un liquide F contenant de l'acide acrylique, selon lequel le liquide F contenant de l'acide acrylique est introduit dans une colonne de rectification à un emplacement d'introduction Z et un courant de matière S est soutiré au-dessus de l'emplacement d'introduction Z de la colonne de rectification à un emplacement de soutirage E, dont la teneur en acide acrylique, par rapport au poids du courant de matière S, est ≥ 90 % en poids et est supérieure à la teneur en acide acrylique correspondante du liquide F en % en poids, **caractérisé en ce que**, dans la zone de la colonne de rectification se trouvant au moins deux étapes de séparation théoriques au-dessus de l'emplacement d'introduction Z, la teneur G du liquide de reflux en acide diacrylique, par rapport au poids du liquide de reflux, est ≥ 550 ppm en poids au moins dans certaines sections.

2. Procédé selon la revendication 1, **caractérisé en ce que** la teneur G du liquide de reflux en acide diacrylique est ≥ 650 ppm en poids.

3. Procédé selon la revendication 1, **caractérisé en ce que** la teneur G du liquide de reflux en acide diacrylique est ≥ 1 000 ppm en poids.

4. Procédé selon l'une quelconque des revendications 1 à 3, **caractérisé en ce que** la teneur G en acide diacrylique du liquide de reflux dans l'ensemble de la zone de la colonne de rectification se trouvant au moins deux étapes de séparation théoriques au-dessus de l'emplacement d'introduction Z est donnée.

5. Procédé selon l'une quelconque des revendications 1 à 4, **caractérisé en ce que** la teneur G en acide diacrylique du liquide de reflux dans l'ensemble de la zone de la colonne de rectification se trouvant au moins quatre étapes de séparation théoriques au-dessus de l'emplacement d'introduction Z est donnée.

6. Procédé selon l'une quelconque des revendications 1 à 5, **caractérisé en ce que** l'emplacement de soutirage E se trouve au moins 2 étapes de séparation théoriques au-dessus de l'emplacement d'introduction Z de la colonne de rectification.

7. Procédé selon l'une quelconque des revendications 1 à 6, **caractérisé en ce que** le liquide F à séparer contient un acide de Bronsted ayant un point d'ébullition inférieur à l'acide acrylique d'un pKₛ ≤ 16 ajouté.

8. Procédé selon l'une quelconque des revendications 1 à 7, **caractérisé en ce que** de l'acide diacrylique en tant que tel et/ou de l'acide acrylique contenant de l'acide diacrylique est ajouté au liquide de reflux.

9. Procédé selon l'une quelconque des revendications 1 à 8, **caractérisé en ce que** la colonne de rectification contient au moins un plateau perforé en tant que composant de séparation.

10. Procédé selon l'une quelconque des revendications 1 à 8, **caractérisé en ce que** la colonne de rectification contient au moins un plateau à courants croisés étanchéifié hydrauliquement en tant que composant de séparation.

11. Procédé selon l'une quelconque des revendications 1 à 10, **caractérisé en ce que** l'énergie thermique nécessaire pour le procédé selon l'invention est introduite par un évaporateur à détente et à circulation forcée extérieur.

12. Procédé selon la revendication 11, **caractérisé en ce que** l'évaporateur à détente et à circulation forcée est un évaporateur tubulaire.

13. Procédé selon l'une quelconque des revendications 1 à 12, **caractérisé en ce que** le liquide F est un mélange qui contient 10 à 35 % en poids d'acide acrylique, 50 à 80 % en poids d'un liquide organique hydrophobe d'un point d'ébullition supérieur à l'acide acrylique et jusqu'à 5 000 ppm en poids d'aldéhydes.

14. Procédé selon l'une quelconque des revendications 1 à 13, **caractérisé en ce que** le liquide F contient les composants suivants :
| | |
|---|---|
| diphyl | 50 à 75 % en poids, |
| phtalate de diméthyle | 10 à 25 % en poids, |
| acide diacrylique | 0,2 à 3 % en poids, |
| acide acrylique | 15 à 35 % en poids, souvent 15 à 25 % en poids, |
| eau | 0,07 à 0,2 % en poids, |
| acide acétique | 0,01 à 0,2 % en poids, |
| acide formique | 0,001 à 0,02 % en poids, |
| acide propionique | 0,001 à 0,02 % en poids, |
| phénothiazine | 0,01 à 0,1 % en poids, |
| anhydride de l'acide | 0,1 à 1 % en poids, |
| phtalique | |
| acide benzoïque | 0,2 à 2 % en poids, |
| anhydride de l'acide | 0,1 à 2 % en poids, |
| maléique | |
| benzaldéhyde | 0,1 à 1 % en poids et |
| furfurals | 0,01 à 0,05 % en poids. |

15. Procédé selon l'une quelconque des revendications 1 à 12, **caractérisé en ce que** le liquide F contient 95 à 99,8 % en poids d'acide acrylique.
